# EUROPÄISCHE PATENTSCHRIFT

(11) **EP 0 688 311 B1**
(45) Veröffentlichungstag und Bekanntmachung des Hinweises auf die Patenterteilung: **21.10.1998**
(21) Anmeldenummer: 94909107.8
(22) Anmeldetag: 03.03.1994
(51) Int. Cl.: C07C 227/40, C07C 231/24, C07C 229/12, C07C 233/36, C11D 1/90

(54) **VERFAHREN ZUR NACHBEHANDLUNG VON BETAINEN UND AMPHOTEREN TENSIDEN**
METHOD OF PROCESSING BETAINES AND AMPHOTERIC SURFACTANTS
PROCEDE PERMETTANT DE RETRAITER DES BETAINES ET DES TENSIOACTIFS AMPHOTERES

(30) Priorität: 12.03.1993 DE 4307791
(43) Veröffentlichungstag der Anmeldung: 27.12.1995
(73) Patentinhaber: Henkel Kommanditgesellschaft auf Aktien, 40191 Düsseldorf (DE)
(72) Erfinder: UPHUES, Günter, D-40789 Monheim (DE); PLOOG, Uwe, D-42781 Haan (DE); JESCHKE, Rainer, D-40595 Düsseldorf (DE); SCHICK, Renate, D-42781 Haan (DE)
(86) Internationale Anmeldenummer: EP9400621
(87) Internationale Veröffentlichungsnummer: WO9420452

(56) Entgegenhaltungen:
- EP-A- 0 020 907
- WO-A-91/08193
- GB-A- 771 082
- US-A- 3 072 690

## Beschreibung

### Gebiet der Erfindung

Die Erfindung betrifft ein Verfahren zur Nachbehandlung von Betainen und amphoteren Tensiden, bei dem man die Tenside alkalisch einstellt und einer Temperaturnachbehandlung unterwirft.

### Stand der Technik

Betaine bzw. amphotere Tenside sind ausgesprochen hautverträglich und weisen ausgezeichnete Reinigungseigenschaften auf. Sie eignen sich daher in besonderer Weise zur Konfektionierung einer Vielzahl von oberflächenaktiven Produkten. Zu ihrer Herstellung geht man im einfachsten Fall von tertiären Aminen aus, die mit Natriumchloracetat zu Alkylbetainen umgesetzt werden. Die Umsetzung von Fettsäureamidoaminen oder Imidazolinen mit Natriumchloracetat führt zur Bildung von amphoteren Tensiden vom Typ der Glycinate; wird als Alkylierungsmittel Acrylsäureester eingesetzt, bilden sich Aminopropionate. Verbindungen der genannten Art sind in einer Vielzahl von Übersichtsartikeln beschrieben, von denen an dieser Stelle nur **Parf.Cosm.Arom. 70, 67 (1986), HAPPI, 70, (Nov.1986)** und **Soap Cosm.Chem.Spec. 46, (Apr.1990)** genannt sein sollen.

Ein besonderes Anliegen bei der Herstellung der Betaine bzw. amphoteren Tenside besteht darin, möglichst reine und somit dermatologisch und toxikologisch unbedenkliche Produkte zur Verfügung zu stellen. Unerwünscht sind beispielsweise Spuren von Chloressigsäure und insbesondere Dichloressigsäure in den Tensiden. Auch Konservierungsstoffe, die die Betaine bzw. amphoteren Tenside vor mikrobiellem Befall schützen sollen, sind häufig nicht erwünscht, so daß ein weiteres Bedürfnis nach Produkten besteht, die auch ohne Zusatz von Hilfsstoffen gegenüber Keimbefall stabilisiert sind. Eine dritte Aufgabe der Erfindung besteht schließlich darin, möglichst hellfarbige Produkte zur Verfügung zu stellen.

Aus dem Stand der Technik sind bereits eine Reihe von Druckschriften bekannt, die Teillösungen für die kumulierte Aufgabenstellung anbieten.

So wird beispielsweise in der **DE-A1 39 39 264** (Henkel) vorgeschlagen, den Gehalt an Chloressigsäure in amphoteren Tensiden durch eine nachträgliche Behandlung der wäßrigen Lösungen mit Ammoniak, Aminosäuren oder Oligopeptiden zu verringern. Aus der **DE-OS 29 26 479** (Th.Goldschmidt) ist ein Verfahren bekannt, bei dem man die Quaternierung im pH-Bereich von 7,5 bis 10,5 durchführt und so den Restgehalt an freiem Alkylierungsmittel minimiert. In die gleiche Richtung weist die Lehre der **DE-A 20 63 424** (Rewo), die die pH-Regulierung für die Alkylierung von Imidazolinen beschreibt. Auf den Gehalt an Dichloressigsäure haben diese Verfahren jedoch keinen Einfluß.

Zur Bleiche oberflächenaktiver Verbindungen werden üblicherweise Natriumhypochlorit oder insbesondere Wasserstoffperoxid eingesetzt. Die Bleiche erfolgt in der Regel in neutralem oder saurem Medium, da sich H₂O₂ unter alkalischen Bedingungen rasch zersetzt. Bei der sauren Peroxidbleiche von Betainen bzw. amphoteren Tensiden stellt man jedoch häufig nur eine temporäre Farbaufhellung fest, die nach Lagerung in eine Farbvertiefung umschlägt.

Aus der Literatur ist ferner ein Verfahren zur konservierungsmittelfreien Stabilisierung von speziellen nichtionischen Tensiden, sogenannten Alkylpolyglucosiden, bekannt, bei dem man die wäßrigen Lösungen auf einen pH-Wert von mindestens 11 einstellt (**DE-A1 40 35 722**, Henkel). Im Hinblick auf die Unterschiede in der Struktur zwischen den nichtionischen Alkylpolyglucosiden und den Betainen bzw. amphoteren Tensiden, die darüber hinaus auch noch eine hydrolysierbare Amidbindung aufweisen, erscheint eine Übertragung des Verfahrens jedoch wenig erfolgversprechend.

### Beschreibung der Erfindung

Gegenstand der Erfindung ist ein Verfahren zur Nachbehandlung von Betainen bzw. amphoteren Tensiden, bei dem man die Tenside durch Zusatz von Alkalihydroxiden auf einen pH-Wert im Bereich von 11 bis 14, vorzugsweise 12 bis 13,5 einstellt und einer Temperaturnachbehandlung, gegebenenfalls unter erhöhtem Druck unterwirft.

Überraschenderweise wurde gefunden, daß eine Temperaturnachbehandlung der Betaintenside im stark alkalischen Milieu zu einer raschen und praktisch quantitativen Abnahme des Dichloressigsäuregehaltes führt. Dies war um so weniger zu erwarten, als daß sich auch Betaine mit einer Amidbindung unter diesen stark alkalischen Bedingungen als äußerst hydrolysestabil erwiesen und selbst bei längerer Lagerung keine signifikante Änderung der Säure- bzw. Aminzahl beobachtet werden konnte. Die Erfindung schließt dabei die Erkenntnis ein, daß die alkalisch eingestellten und nachbehandelten Produkte gegenüber Keimbefall hervorragend stabilisiert sind, so daß eine zusätzliche Konservierung nicht mehr erforderlich ist. Schließlich wurde die überraschende Beobachtung gemacht, daß die nach dem erfindungsgemäßen Verfahren erhaltenen, stark alkalisch eingestellten Produkte einer Peroxidbleiche sehr leicht zugänglich sind und unerwartet hellfarbige Produkte erhalten werden können.

### Betaine bzw. amphotere Tenside

Grundsätzlich kann das erfindungsgemäße Verfahren auf alle Betaine und amphoteren Tenside angewendet werden. Bei solchen Typen, die nicht über die Alkylierung mit Natriumchloracetat hergestellt werden - beispielsweise Aminopropionate oder Sulfobetaine - kann naturgemäß der Vorteil einer Abtrennung von Dichloressigsäure nicht zum Tragen kommen, die Temperaturnachbehandlung der stark alkalisch eingestellten Produkte bewirkt jedoch auch in diesen Fällen eine Stabilisierung gegen Keimbefall und macht die Tenside einer Peroxidbleiche leichter zugänglich.

Vorzugsweise kann das erfindungsgemäße Verfahren jedoch auf Beteine vom Typ der Alkylbetaine angewendet werden, die der Formel (I) folgen, 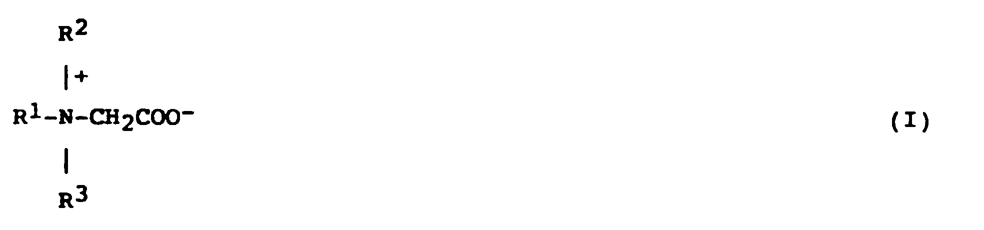 in der R¹ für einen Alkyl- und/oder Alkenylrest mit 6 bis 22 Kohlenstoffatomen und R² und R³ unabhängig voneinander für einen Alkyl- und/oder Hydroxyalkylrest mit 1 bis 4 Kohlenstoffatomen steht.

Typische Beispiele stellen Umsetzungsprodukte von tertiären Aminen, insbesondere Dimethylalkylaminen mit Natriumchloracetat dar.

Eine weitere bevorzugte Gruppe von Einsatzstoffen stellen die **Glycinate bzw. Fettsäureamido-N-N-dialkylbetaine** dar, die der Formel (II) folgen, 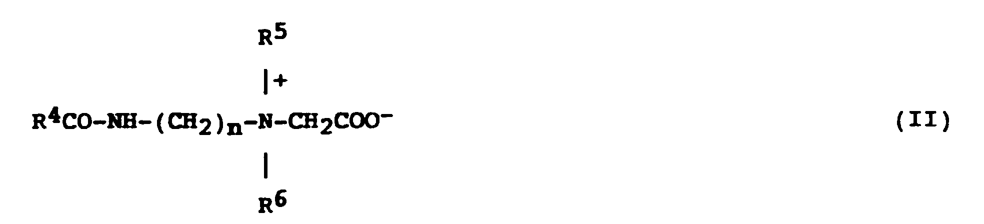 in der R⁴CO für einen gesättigten und/oder ungesättigten Acylrest mit 6 bis 22 Kohlenstoffatomen, R⁵ für Wasserstoff oder einen Alkyl- und/oder Hydroxyalkylrest mit 1 bis 4 Kohlenstoffatomen, R⁶ für einen Alkyl- und/oder Hydroxyalkylrest mit 1 bis 4 Kohlenstoffatomen und n für 2 oder 3 steht. Vorzugsweise werden Betaine der Formel (II) in das erfindungsgemäße Verfahren eingesetzt, in der R⁴CO für einen Acylrest mit 8 bis 18 Kohlenstoffatomen, R⁵ und R⁶ für eine Methyl- und/oder Hydroxyethylgruppe und n für 3 steht.

### Thermische Nachbehandlung

Eine wesentliche Voraussetzung für das erfindungsgemäße Verfahren besteht in der Einstellung des pH-Wertes. Üblicherweise erfolgt die Regulierung durch Zugabe eines Alkalihydroxids zu den wäßrigen Pasten oder Lösungen der Betaine bzw. amphoteren Tenside. Die Alkalihydroxide können in Form von 5 bis 55, vorzugsweise 25 bis 50 gew.-%iger wäßriger Lösungen eingesetzt werden. Vorzugsweise werden konzentrierte, d. h. ca. 50 gew.-%ige Natriumhydroxidlösungen eingesetzt.

Anschließend werden die alkalisch eingestellten Betaine bzw. amphoteren Tenside einer Temperaturnachbehandlung unterworfen. Die Nachbehandlung kann bei Temperaturen von 20 bis 130°C beispielsweise in einem Rührkessel durchgeführt werden. In einer bevorzugten Ausführungsform des erfindungsgemäßen Verfahrens erfolgt die Nachbehandlung jedoch in einem Druckbehälter bei Temperaturen im Bereich von 90 bis 120°C, gegebenenfalls bei erhöhtem autogenen Druck im Bereich von ca. 1 bis 2 bar. Die Temperaturnachbehandlung dient primär dem Zweck, den Gehalt an Dichloressigsäure zu erniedrigen, wozu es einer Reaktionszeit im Bereich von 0,1 bis 5, vorzugsweise 1 bis 3 h bedarf. Gleichzeitig wird dabei auch der Gehalt an eventuell vorhandener freier Monochloressigsäure signifikant, d. h. bis unterhalb der instrumentellen Nachweisgrenze herabgesetzt.

Besteht das Ziel hingegen allein darin, eine ausreichende Stabilisierung gegen Keimbefall sicherzustellen und die Bleichbarkeit der Tenside zu verbessern, ist es ausreichend, die Produkte bei Raumtemperatur stark alkalisch einzustellen und unmittelbar weiterzubehandeln, beispielsweise durch Zusatz von 0,01 bis 1, vorzugsweise 0,1 bis 0,5 Gew.-% Wasserstoffperoxid - bezogen auf den Feststoffgehalt der Produkte - bei Temperaturen im Bereich von 50 bis 95, vorzugsweise 60 bis 90°C zu bleichen.

Im Anschluß an die Temperaturbehandlung können die Produkte - falls gewünscht - beispielsweise durch Zugabe von Mineralsäure auf einen pH-Wert im Bereich von 7 bis 5 eingestellt werden. Bei dieser Maßnahme geht freilich der Schutz gegen mikrobiellen Befall verloren.

### Gewerbliche Anwendbarkeit

Die nach dem erfindungsgemäßen Verfahren erhältlichen Produkte weisen einen äußerst niedrigen Restgehalt an Dichloressigsäure auf und sind ohne Zusatz von Konservierungsmitteln gegen mikrobiellen Befall stabilisiert. Sie eignen sich daher zur Herstellung von oberflächenaktiven Mitteln, beispielsweise Wasch-, Spül- und Reinigungsmitteln, sowie Produkten zur Haar- und Körperpflege, in denen sie in Mengen von 1 bis 30, vorzugsweise 2 bis 10 Gew.-% - bezogen auf die Mittel - enthalten sein können.

Die folgenden Beispiele sollen den Gegenstand der Erfindung näher erläutern, ohne ihn darauf einzuschränken.

### Beispiele

### I. Hydrolysestabilität

C_{8/18}-Kokosfettsäureamidopropyl-N,N-dimethylaminobetain (Dehyton^{(R)} K, Verkaufsprodukt Fa.Henkel KGaA, Düsseldorf/ FRG, Feststoffgehalt ca. 35 Gew.-%) wurde durch Zugabe von wäßriger Natriumhydroxidlösung auf pH 11 eingestellt und bei einer Temperatur von 20 bzw. 40°C über einen Zeitraum von 8 Wochen gelagert. Während dieser Zeit wurden der Verlauf des pH-Wertes sowie die Säure- und Aminzahlen überwacht. Die Ergebnisse sind in Tab.1 zusammengefaßt:

**Tab.1:**

| Lagerversuche mit Dehyton^{(R)} K | | | | | | | |
|---|---|---|---|---|---|---|---|
| Bsp. | t | pH-Wert | | SZ | | AZ | |
| | | 20°C | 40°C | 20°C | 40°C | 20°C | 40°C |
| 1 | 0 | 11,00 | 11,00 | 3,0 | 3,0 | 0,50 | 0,50 |
| 2 | 1 d | 10,89 | 11,31 | 2,7 | 3,1 | 0,47 | 0,53 |
| 3 | 3 d | 10,74 | 11,24 | 2,7 | 3,1 | 0,50 | 0,50 |
| 4 | 5 d | 10,66 | 11,24 | 2,6 | 2,9 | 0,50 | 0,50 |
| 5 | 2 w | 10,46 | 10,96 | 2,9 | 2,9 | 0,47 | 0,47 |
| 6 | 3 w | 10,42 | 10,92 | 2,9 | 2,8 | 0,47 | 0,50 |
| 7 | 4 w | 10,20 | 10,90 | 2,9 | 2,8 | 0,50 | 0,47 |
| 8 | 8 w | 10,20 | 10,88 | 2,9 | 3,0 | 0,50 | 0,53 |
| Legende: t = Lagerzeit SZ = Säurezahl AZ = Aminzahl | | | | | | | |

Die Beispiele zeigen, daß auch unter extremen Lagerbedingungen (pH 11, 40°C, 8 Wochen) Säure- und Aminzahl konstant bleiben und eine Hydrolyse der Amidbindung nicht stattfindet.

### II. Bleiche

C_{8/18}-Kokosfettsäureamidopropyl-N,N-dimethylaminobetain (Dehyton^{(R)} K, Verkaufsprodukt Fa.Henkel KGaA, Düsseldorf/FRG, Feststoffgehalt ca. 35 Gew.-%) wurde bei unterschiedlichen pH-Werten mit 0,1 bis 1 Gew.-% Wasserstoffperoxid (30 gew.-%ig) - bezogen auf den Feststoffgehalt - versetzt und 30 min bei 60°C gebleicht. Die Ergebnisse sind in Tab.2 zusammengefaßt:

**Tab.2:**

| Bleiche von Dehyton^{(R)} K | | | |
|---|---|---|---|
| Bsp. | pH-Wert | c(H₂O₂) | Farbzahl |
| | | Gew.-% | APHA |
| 9 | 11 | 0,1 | 35 |
| 10 | 12 | 0,1 | 40 |
| 11 | 12 | 1,0 | 30 |
| 12 | 13 | 0,1 | 50 |
| V1 | 7 | - | 100 |
| V2 | 5 | 0,1 | 120 |
| V3 | 3 | 0,1 | 125 |
| V4 | 9 | 0,1 | 95 |
| Legende: c(H₂O₂) = Konezntration Bleichmittel | | | |

### III. Dichloressigsäuregehalt

C_{8/18}-Kokosfettsäureamidopropyl-N,N-dimethylaminobetain (Dehyton^{(R)} K, Verkaufsprodukt Fa.Henkel KGaA, Düsseldorf/ FRG, Feststoffgehalt ca. 35 Gew.-%) wurde durch Zugabe von wäßriger Natriumhydroxidlösung auf pH = 12,5 bis 13,5 - bezogen auf 10 gew.-%ige Produktlösungen bei 20°C - eingestellt und im Autoklaven bei einer Temperatur von 90 bis 120°C und einem Druck von 1 bis 1,1 bar über einen Zeitraum von 0,5 bis 3 h nachbehandelt. Die Ergebnisse zum Gehalt an Dichloressigsäure sind in Tab.3 zusammengefaßt:

**Tab.3:**

| Dichloressigsäuregehalt in Dehyton^{(R)} K | | | | | | |
|---|---|---|---|---|---|---|
| Bsp. | pH-Wert | T | p | t | c(DCE) | c(Alk) |
| | | °C | bar | h | ppm | mmg |
| V4 | 7,0 | | | | 97 | 90,7 |
| V5 | 9,0 | 92 | 1,0 | 3,0 | 88 | 95,5 |
| V6 | 10,5 | 92 | 1,0 | 3,0 | 86 | 98,4 |
| 13 | 13,0 | 92 | 1,0 | 0,5 | 68 | 129,3 |
| | 13,0 | 92 | 1,0 | 1,0 | 61 | 129,3 |
| | 13,0 | 92 | 1,0 | 2,0 | 49 | 129,3 |
| | 13,0 | 92 | 1,0 | 3,0 | 38 | 129,3 |
| 14 | 13,5 | 92 | 1.0 | 0,5 | 62 | 132,9 |
| | 13,5 | 92 | 1,0 | 1,0 | 60 | 132,9 |
| | 13,5 | 92 | 1,0 | 2,0 | 46 | 132,9 |
| | 13,5 | 92 | 1,0 | 3,0 | 34 | 132,9 |

**Forts.Tab.3:**

| Dichloressigsäuregehalt in Dehyton^{(R)} K | | | | | | |
|---|---|---|---|---|---|---|
| Bsp. | pH-Wert | T | p | t | c(DCE) | c(Alk) |
| | | °C | bar | h | ppm | mmg |
| 15 | 13,5 | 120 | 1,1 | 1,0 | 5 | 136,4 |
| | 13,5 | 120 | 1,1 | 2,0 | 5 | 136,4 |
| | 13,5 | 120 | 1,1 | 3,0 | 2 | 136,4 |
| 16 | 12,5 | 120 | 1,1 | 1,0 | 25 | 114,3 |
| | 12,5 | 120 | 1,1 | 2,0 | 5 | 114,3 |
| Legende: T = Nachbehandlungstemperatur t = Nachbehandlungszeit p = Druck c(DCE) = Gehalt Dichloressigsäure (via HPLC) c(Alk) = Gsamtalkaligehalt nach Titration mit Perchlorsäure mmg = mmol/100 g | | | | | | |

## Patentansprüche

1. Verfahren zur Nachbehandlung von Betainen bzw. amphoteren Tensiden, bei dem man die Tenside durch Zusatz von Alkalihydroxiden auf einen pH-Wert im Bereich von 11 bis 14 einstellt und einer Temperaturnachbehandlung, gegebenenfalls unter erhöhtem Druck unterwirft.

2. Verfahren nach Anspruch 1, **dadurch gekennzeichnet**, daß man als Tenside Alkylbetaine der Formel (I) einsetzt, 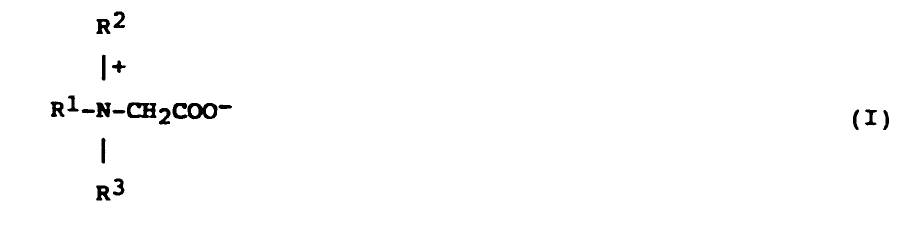 in der R¹ für einen Alkyl- und/oder Alkenylrest mit 6 bis 22 Kohlenstoffatomen und R² und R³ unabhängig voneinander für einen Alkyl- und/oder Hydroxyalkylrest mit 1 bis 4 Kohlenstoffatomen steht.

3. Verfahren nach Anspruch 1, **dadurch gekennzeichnet**, daß man als Tenside Verbindungen der Formel (II) einsetzt, 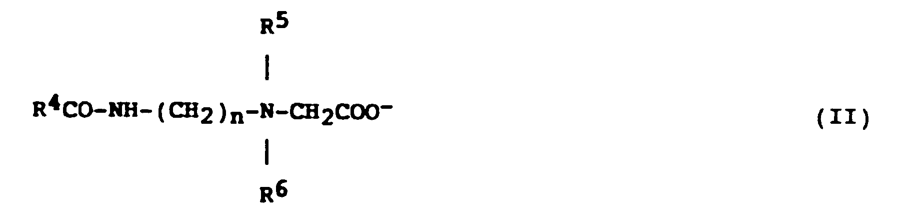 in der R⁴CO für einen gesättigten und/oder ungesättigten Acylrest mit 6 bis 22 Kohlenstoffatomen, R⁵ für Wasserstoff oder einen Alkyl- und/oder Hydroxyalkylrest mit 1 bis 4 Kohlenstoffatomen, R⁶ für einen Alkyl- und/oder Hydroxyalkylrest mit 1 bis 4 Kohlenstoffatomen und n für 2 oder 3 steht.

4. Verfahren nach den Ansprüchen 1 bis 3, **dadurch gekennzeichnet**, daß man als Alkalihydroxid Natriumhydroxid einsetzt.

5. Verfahren nach den Ansprüchen 1 bis 4, **dadurch gekennzeichnet**, daß man die Nachbehandlung der Betaine bzw. amphoteren Tenside bei Temperaturen im Bereich von 20 bis 130°C und bei Drücken im Bereich von 1 bis 2 bar durchführt.

6. Verfahren nach den Ansprüchen 1 bis 5, **dadurch gekennzeichnet**, daß man die Betaine bzw. amphoteren Tenside im Anschluß an die Temperaturnachbehandlung mit Wasserstoffperoxid bleicht.

7. Verfahren nach den Ansprüchen 1 bis 6, **dadurch gekennzeichnet**, daß man die Betaine bzw. amphoteren Tenside im Anschluß an die Temperaturnachbehandlung auf einen pH-Wert im Bereich von 7 bis 5 einstellt.

## Claims

1. A process for the aftertreatment of betaines and amphoteric surfactants, in which the surfactants are adjusted to a pH value of 11 to 14 by addition of alkali metal hydroxides and subjected to a thermal aftertreatment, optionally under elevated pressure.

2. A process as claimed in claim 1, **characterized in that** alkyl betaines corresponding to formula (I): 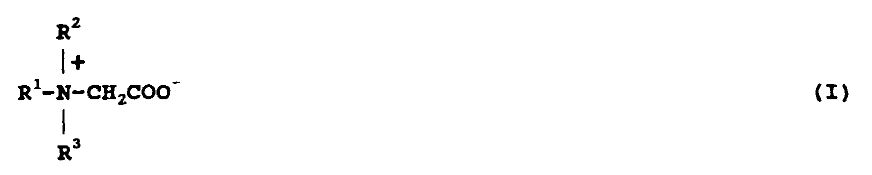 in which R¹ is an alkyl and/or alkenyl radical containing 6 to 22 carbon atoms and R² and R³ independently of one another represent an alkyl and/or hydroxyalkyl radical containing 1 to 4 carbon atoms,
are used as surfactants.

3. A process as claimed in claim 1, **characterized in that** compounds corresponding to formula (II): 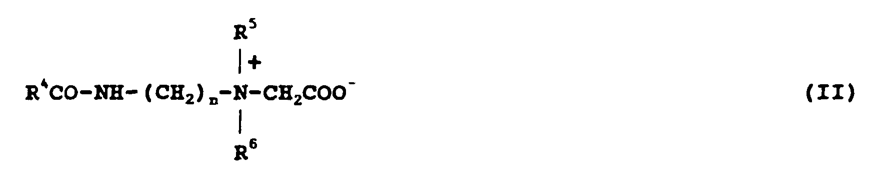 in which R⁴CO is a saturated and/or unsaturated acyl radical containing 6 to 22 carbon atoms, R⁵ is hydrogen or an alkyl and/or hydroxyalkyl radical containing 1 to 4 carbon atoms, R⁶ is an alkyl and/or hydroxyalkyl radical containing 1 to 4 carbon atoms and n = 2 or 3, are used as surfactants.

4. A process as claimed in claims 1 to 3 **characterized in that** sodium hydroxide is used as the alkali metal hydroxide.

5. A process as claimed in claims 1 to 4, **characterized in that** the aftertreatment of the betaines or amphoteric surfactants is carried out at temperatures of 20 to 130°C under pressures of 1 to 2 bar.

6. A process as claimed in claims 1 to 5, **characterized in that** the betaines or amphoteric surfactants are bleached with hydrogen peroxide after the thermal aftertreatment.

7. A process as claimed in claims 1 to 6, **characterized in that** the betaines or amphoteric surfactants are adjusted to a pH value of 7 to 5 after the thermal aftertreatment.

## Revendications

1. Procédé de retraitement de bétaïnes ou de tensioactifs amphotères, dans lequel on ajuste le pH des tensioactifs par adjonction d'hydroxydes de métaux alcalins à un valeur comprise dans l'intervalle de 11 à 14 et on les soumet à un retraitement thermique, éventuellement sous pression accrue.

2. Procédé selon la revendication 1, **caractérisé en ce que** l'on met en oeuvre comme tensioactifs, des alkylbétaïnes de la formule (I), 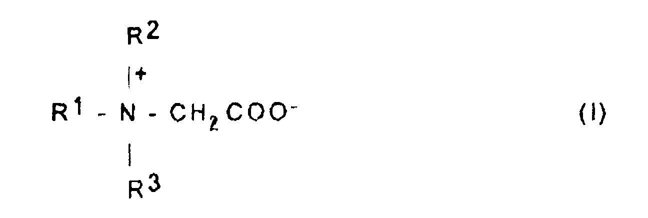 dans laquelle R¹ représente un radical alkyle et/ou alcényle comportant 6 à 22 atomes de carbone, et R² et R³ correspondent indépendamment l'un de l'autre à un radical alkyle et/ou hydroxyalkyle comportant 1 à 4 atomes de carbone.

3. Procédé selon la revendication 1, **caractérisé en ce que** l'on met en oeuvre comme tensioactifs, des composés de la formule (II) 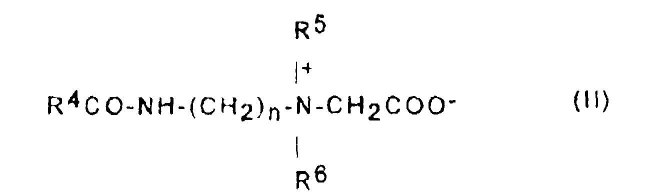 dans laquelle R⁴CO représente un radical acyle saturé et/ou insaturé comportant 6 à 22 atomes de carbone, R⁵ correspond à l'hydrogène ou à un radical alkyle et/ou hydroxyalkyle possédant 1 à 4 atomes de carbone, R⁶ est un radical alkyle et/ou hydroxyalkyle présentant 1 à 4 atomes de carbone, et n est égal à 2 ou à 3.

4. Procédé selon les revendications 1 à 3, **caractérisé en ce que** l'on met en oeuvre de l'hydroxyde de sodium comme hydroxyde de métal alcalin.

5. Procédé selon les revendications 1 à 4, **caractérisé en ce que** l'on opère le retraitement des bétaïnes ou des tensioactifs amphotères à des températures dans la plage de 20 à 130 °C et à des pressions dans l'intervalle de 1 à 2 bars,

6. Procédé selon les revendications 1 à 5, **caractérisé en ce que** l'on blanchit les bétaînes ou les tensioactifs amphotères au moyen d'eau oxygénée, à la suite du retraitement thermique.

7. Procédé selon les revendications 1 à 6, **caractérisé en ce que** l'on ajuste le pH des bétaïnes ou des tensioactifs amphotères à un pH dans la plage de 7 à 5, à la suite du retraitement thermique.
